# EUROPEAN PATENT APPLICATION

(11) **EP 2 143 448 A1**
(43) Date of publication of application: **13.01.2010**
(21) Application number: 09001290.7
(22) Date of filing: 30.01.2009
(51) Int. Cl.: A61L 9/12, B60H 3/00

(54) **Improved essential oil diffuser for motor vehicles**

(30) Priority: 09.07.2008 IT MI20081241
(71) Applicant: TAVOLA S.p.A., 20141 Milano (IT)
(72) Inventor: Cavallo, Adriano, 20089 Rozzano Milano (IT)
(74) Representative: La Ciura, Salvatore

(57) **Abstract**

An essential oil diffuser for motor vehicles of the type adapted to be placed on the air vents, having an outer shell which receives a bottle containing the essential oil to be diffused in the interior compartment by the air stream coming from the vents, which flows over a wick soaked in said bottle and carries a certain amount of evaporated essential oil in suspension.

## Description

The present invention provides an essential oil diffuser for motor vehicles, of the type adapted to be placed on air grilles, having a cylindrical shell, which receives a bottle containing the scented oil and has a top opening for conveying the air stream coming from the vents onto a wick soaked in the oil to dispense the scent thereof, **characterized in that** it has means for improving the diffuser efficiency when low-temperature air conditioning is on.

In greater detail, the invention provides the use of a movable partition which is adapted to translate from a lowered position in which the top opening of the shell is clear, to a lifted position in which said partition partially obstructs this air stream passage and particularly prevents cold air coming out of the vent to directly impinge upon the soaked wick, thereby inhabiting the oil scenting effect.

The partition causes some slight turbulence, thereby delaying the ingress of air into the shell so that by coming in contact for a moment with the air of the interior compartment, its temperature is increased before impinging upon the soaked wick.

The partition may be simply and quickly lowered or lifted by the driver as needed, without removing the diffuser.

A variety of scenters and fragrance dispensers for motor vehicles are currently available.

Some of them are sheets of cardboard, cellulose or similar materials soaked with a given amount of scented oil, which may be placed in the interior compartment in a desired position, such as hanging from the rear view mirror or passenger handles.

Nevertheless, the disadvantage of these products is that they do not allow control of the amount of essential oil released in the car interior.

Particularly, these scenters release a very strong, sometimes even offensive fragrance in the first days after application, which grows fainter rather quickly, thereby requiring frequent scenter replacements.

Another type of diffusers is mounted to the air grilles of the vehicles, and releases a substantially constant amount of fragrance with time.

These devices are composed of an outer shell of various shapes and sizes, which receives a bottle containing a scented material to be diffused in the interior compartment by the air stream coming from said air grilles which flows over a wick soaked in the bottle and carries a small amount of evaporated scented material.

These prior art devices also suffer from certain drawbacks.

As is known, the scent strength changes with the temperature of the air stream that flows over the wick and particularly decreases as temperature decreases, because a smaller amount of liquid evaporates and is carried by air in suspension.

Therefore, a problem arises in summertime, when driving with air conditioning on, because the air stream from the vents has a very low temperature (about 5°C - 6°C) which inhibits the scenting effect of the diffuser.

In an attempt to obviate the above drawbacks, the present invention provides an essential oil diffuser for motor vehicles, of the type adapted for application to the air vents, **characterized in that** it has means for preventing the air stream coming out of said vents from directly impinging against the soaked wick and for creating a slight turbulence which allows it to be mixed with ambient air and to significantly increase its temperature, prior to its ingress into the diffuser body.

These means consist of a vertically sliding partition, which may be manually operated by the driver when air conditioning is on.

By partially obstructing the air stream opening, said partition slightly deflects the stream thereby delaying its ingress into the shell and allowing cold air to mix with the interior compartment air and increase its temperature.

The present invention will now be described by way of example and without iimitation, with reference to the accompanying drawings, in which:

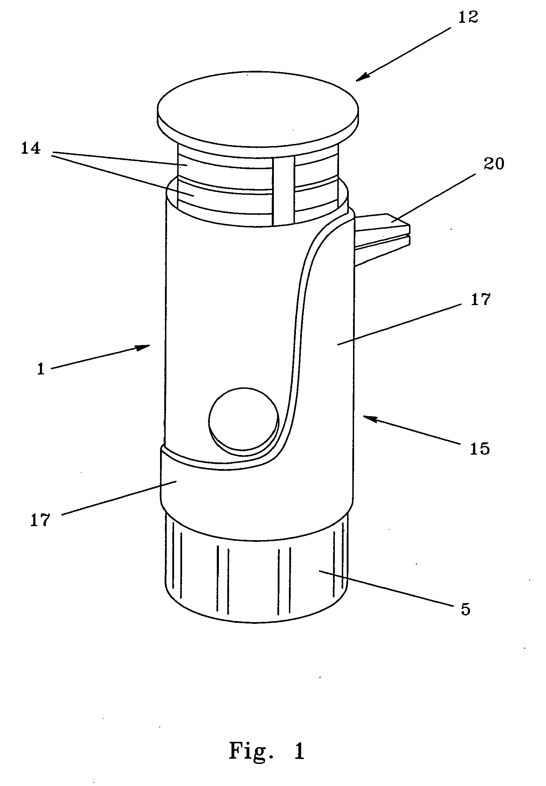

Figure 1 is a perspective view of the diffuser of the invention with the partition in the lowered position.

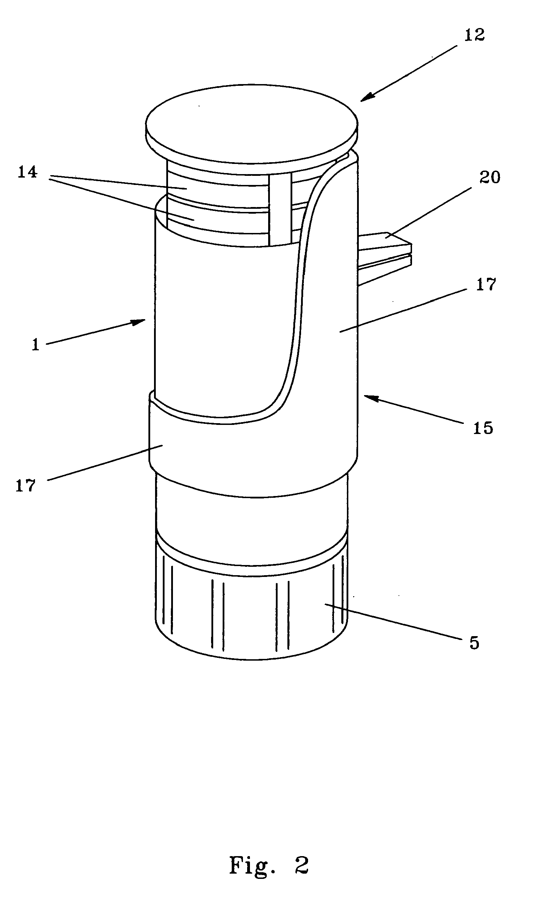

Figure 2 is a perspective view of the scenter of the invention with the partition in the lifted position.

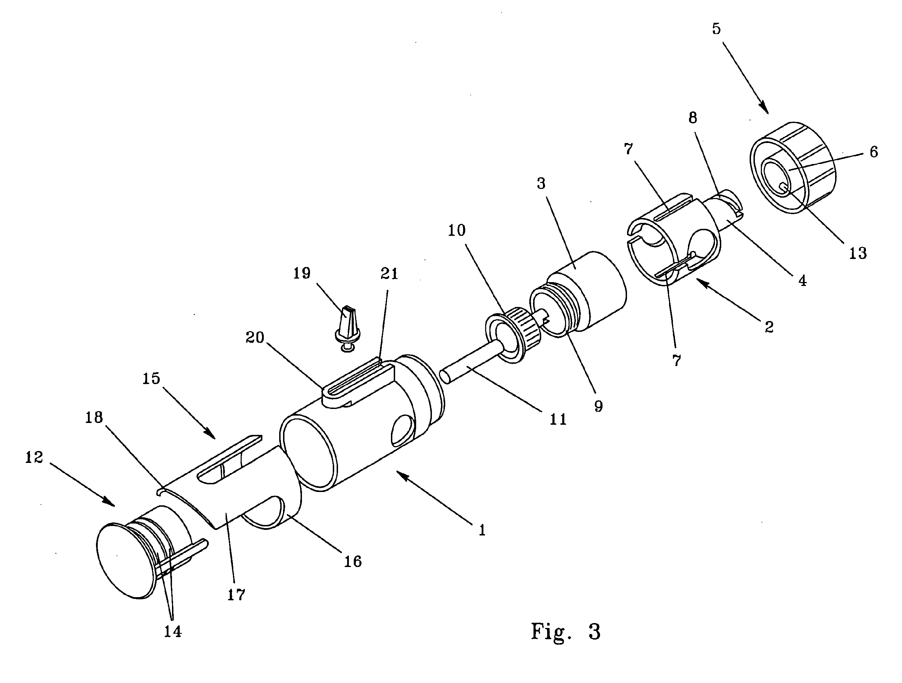

Figure 3 is a perspective exploded view of the diffuser.

Referring to the annexed figures, the essential oil diffuser for motor vehicles has a hollow cylindrical outer shell 1, which is adapted to contain a second movable shell 2 axially sliding in the shell 1, in which an essential oil bottle 3 is received.

This movable shell 2 has a cylindrical end 4 at its bottom, which is received in a housing 6, also having a circular shape, formed in the bottom knob 5 and two slots 7 for receiving a pair of pins (not shown) guiding the translation of the shell 2 in the shell 1.

A projecting pin 13 is provided on the inner surface of said housing 6 and is designed to be received in a helical guide 8 formed on the outer surface of the end 4 of the shell 2.

A hollow cylindrical wick-supporting ring nut 10 is received in the neck 9 of the bottle 3, and in turn receives the wick 11 with the bottom in contact with the liquid and the top projecting a few millimeters out of the edge of the ring nut 10.

The bottle 3 is closed at its top by a screw cap 12 which is tightened onto the neck 9 and has apertures 14 on its lateral surface at the portion of the wick 11 that projects out of the ring nut 10.

The shell 2, the bottle 3 and the cap 12 are integrally formed and contained in the shell 1.

The knob 5 is axially restrained on the shell 1 by means of grooved profiles (not shown), and is rotatable relative to it; thus, by rotating said knob 5, the pin 7 slides into the helical guide 8, thereby causing the movable shell 2, the bottle 3 and the cap 11 to translate into the shell 1.

More in detail, the knob 5 can rotate to gradually move the cap 11 from a closed position, in which it is entirely contained in the shell 1, to an open position in which said cap projects out of the top of the shell 1 thereby exposing the wick 11 through the apertures 14.

By this adjustment, the scent strength can be controlled by varying the air stream that enters the apertures 14 and impinges against the wick 11.

An external sleeve 15 is provided outside the shell 1, which comprises a circular collar 16 surrounding the cylindrical shell 1, with a partition 17 extending therefrom along an angular portion of said collar towards the rear of the diffuser (i.e. the part adjacent to the air grilles).

This sleeve 15 is able to vertically translate from a lowered position, in which the upper edge 18 of the partition 17 remains substantially below the top of the shell 1, to a lifted position in which said partition 17 may project cut of top of the shell 1.

The diffuser is attached to the fins of the air grille by a rear fork 19 which is received in an undercut housing 20 formed in the projecting element 21.

The operation is as follows:
when the diffuser is not intended to be operated, the knob 5 is rotated into the closed position, in which the cap 11 is entirely contained in the shape of the shell 1 and the wick does not contact the air of the interior compartment.

The diffuser may be simply operated by rotating the knob 5 so that a section of the cap 11 is caused to project, according to the desired scent strength.

Thus, the air stream coming from the vents penetrates the apertures 14 facing towards the air grilles and flows over the internal soaked wick 11 and carries atomized scented oil particles.

When low-temperature air conditioning is turned on, the sleeve 15 will be simply lifted to move the partition 17 to the projecting part of the cap 11.

Hence, the air stream does not directly impinge against the wick 11 but is deflected sideways and flows into the cap 11 through the front (i.e. the part facing towards the interior compartment).

In the short path covered by the air stream, the temperature of the latter is increased because of the generated turbulence and to its being mixed with the warmer air of the interior compartment.

The higher temperature of air enhances evaporation of the liquid soaking the wick thereby increasing the scent strength as compared with prior art devices.

Therefore, the present invention provides an essential oil diffuser for motor vehicles that can maintain its scent efficiency unchanged even when the car air conditioner is on.

Those skilled in the art will appreciate that many changes and variants may be made to the above, without departure from the scope of the present invention.

## Claims

1. An essential oil diffuser for motor vehicles of the type adapted to be placed on the air vents, having an outer shell which receives a bottle containing the essential oil to be diffused in the interior compartment by the air stream coming from the vents, which flows over a wick soaked in said bottle and carries a certain amount of evaporated essential oil in suspension, **characterized in that** it has means for deflecting the air stream coming out of the vents, so that it can mix for a moment with the interior compartment air, before impinging against the wick.

2. An essential oil diffuser for motor vehicles as claimed in claim 1, **characterized in that** said means consist of a partition that is able to vertically slide between a lowered position in which it does not interfere with the air flow coming from the air vents to the wick, and a lifted position in which it is interposed between the air vent and the wick and prevents the air stream from directly impinging upon the wick.

3. An essential oil diffuser for motor vehicles as claimed in claim 1, **characterized in that** it comprises:
- an outer shell;
- an inner shell axially sliding in the outer shell;
- a bottle containing the wick-soaking essential oil, which is received in the inner shell;
- a top cap screwed onto the bottle neck, having apertures at the portion of the wick that projects out of the bottle;
- a bottom knob axially restrained with the outer shell, whose rotation causes the inner shell, the bottle and the top cap to translate into the outer shell;
- a partition vertically sliding on the outer shell, which is located at the rear of the diffuser, i.e. the part facing towards the vents.

4. An essential oii diffuser for motor vehicles as claimed in any preceding claim, **characterized in that** said partition is connected to an annular collar which encircles the outer shell and slides vertically relative to it.

5. An essential oil diffuser for motor vehicles as claimed in any preceding claim, **characterized in that** it has a screw system or the like for allowing vertical translation of the inner she!!, the bottle and the top cap, by rotating the bottom knob.
